Europäisches Patentamt

(19) European Patent Office

Office européen des brevets

(11) Publication number: **0 328 507 B1**

(12) # EUROPEAN PATENT SPECIFICATION

(45) Date of publication of patent specification:
**19.06.91 Bulletin 91/25**

(21) Application number: **89870026.5**

(22) Date of filing: **10.02.89**

(51) Int. Cl.⁵: **B01J 23/62**, B01J 37/00,
C07C 5/32, C07C 5/41,
C07B 35/02

(54) **Process for preparing catalysts containing a metal of the platinum group, and uses thereof.**

(30) Priority: **11.02.88 BE 8800157**
**11.02.88 BE 8800158**

(43) Date of publication of application:
**16.08.89 Bulletin 89/33**

(45) Publication of the grant of the patent:
**19.06.91 Bulletin 91/25**

(84) Designated Contracting States:
**AT BE CH DE ES FR GB GR IT LI LU NL SE**

(56) References cited:
**EP-A- 0 020 240**
**EP-A- 0 100 222**
**US-A- 3 649 565**
**US-A- 3 880 776**
**US-A- 3 909 451**
**US-A- 4 133 839**

(73) Proprietor: **FINA RESEARCH S.A.**
**Zone Industrielle C**
**B-6520 Seneffe (Feluy) (BE)**

(72) Inventor: **De Clippeleir, Georges**
**Petrus Huysegomstraat 5**
**B-1600 Sint-Pieters-Leeuw (BE)**
Inventor: **Baumann Ofstad, Elizabeth**
**Sandbunnveien 11**
**N-1342 Jar (NO)**
Inventor: **Kvisle, Steinar**
**Hegermannsgt 2-1**
**N-0478 Oslo 4 (NO)**

(74) Representative: **Leyder, Francis**
**c/o Fina Research S.A. Dépt. Brevets Zone**
**Industrielle C**
**B-6520 Feluy (BE)**

## Description

The present invention relates to a process for preparing catalysts containing at least one metal of the platinum group, in order to obtain more effective or performing catalysts in various types of reactions such as dehydrogenation, hydrogenation or dehydrocyclisation of hydrocarbons, more particularly for the dehydrogenation of paraffinic hydrocarbons having from 3 to 6 carbon atoms.

Many methods exist to prepare supported catalysts. In the present case, the best known catalysts are constituted by an alumina support which is impregnated with a metallic compound of the tin group and with a metallic compound of the platinum group. Generally, the catalysts may also contain an alkaline or alkaline-earth promoter. The usual methods to prepare those catalysts comprise successive impregnations which are separated by either a drying step or a calcination step, although they sometimes consisted of a single impregnation step.

However, the so-formed catalysts are still not sufficiently performing, particularly when used in hydrocarbon hydrogenation or dehydrogenation processes.

The catalytic dehydrogenation of hydrocarbons may be achieved since many years and constitutes an important catalytic process in view of the increasing request for dehydrogenated products which may be valorized under various forms, such as gasolines having a high octane number, plastic materials and synthetic rubbers.

Moreover, in view of the increasing production of LPG (liquified petroleum gas), it seems particularly interesting to convert a part of those paraffinic hydrocarbons into dehydrogenated products such as propylene which is particularly interesting for the manufacture of plastic materials.

The catalytic dehydrogenation of hydrocarbons is carried out in the presence of catalytic compositions containing platinum deposited on a support. The catalytic compositions may also contain other catalytically active metals such as tin or indium and promoters based on alkaline or alkaline-earth metals.

Such processes and catalytic compositions have been described e.g. in US Patents 2,479,209, 2,602,772, 2,930,763, 3,531,543, 3,745,112, 3,892,657, 3,909,451 or still in US Patents 4,329,258, 4,363,721 and 4,430,517.

Further, in most of these patents, it is also taught to use a halogen in order to improve the yield of the dehydrogenation reaction.

However, despite all these already proposed developments, when they are applied to the dehydrogenation of propane, the yield of the reaction at 600°C does not exceed about 30 to 35%, i.e. 70 to 81% with regard to the theoretical conversion, taking into account the thermodynamic constraints.

There is therefore a need to provide a process for preparing catalysts containing a metal of the platinum group and a metal of the tin group, which enables to obtain more active catalysts.

The object of the present invention is to provide an improved process for preparing catalysts containing at least one metal of the platinum group and at least one metal of the tin group together with a promoter, said catalyst having a more important activity than similar catalysts prepared according to usual methods.

Another object of the present invention is to provide an improved process for the dehydrogenation of paraffinic hydrocarbons having from 3 to 6 carbon atoms in the presence of a catalyst containing at least one metal selected from the group of platinum, a metal selected from the group of tin and a promoter.

A further object of the present invention is to provide an improved process for the dehydrogenation of propane into propylene in the presence of a catalyst containing one or more metal selected from the platinum group, a metal selected from the tin group and a promoter of the alkaline or alkaline-earth metal type, the deposition of the one or more metal of the platinum group on the catalyst being carried out in two steps.

The process of the present invention for the preparation of a catalyst consisting of an alumina support containing at least one metal from the platinum group together with a co-catalyst and a promoter comprises the step of submitting the alumina support, containing the co-catalyst and calcined at a temperature comprised between about 450 and about 550°C,

    – to a first treatment with a compound of metal of the platinum group, said first treatment being followed by a calcination in air and by a reduction in the presence of hydrogen at a temperature comprised between 450 and 550°C ;

    – then to an intermediate treatment to deposit the promoter, said intermediate treatment being followed by a calcination at a temperature comprised between about 380 and about 550°C ;

    – and finally to a second treatment with a compound of metal of the platinum group, said second treatment being followed by a calcination at a temperature not exceeding 525°C,

in order to obtain a catalyst containing from 0.1 to 2% by weight of at least one metal from the platinum group, from 0.1 to 2% by weight of at least one metal from the tin group as co-catalyst and from 0.5 to 5% by weight of at least one alkaline or alkaline-earth metal as promoter.

The catalyst used in the present invention comprises an alumina support wherein are introduced at least

2

a metal of the platinum group (as herein defined and referred to as the metal of the platinum group), a metal of the tin group (as herein defined and referred to as the metal of the tin group) and a promoter of the alkaline or alkaline-earth metal type (as herein defined and referred to as the promoter). The metal of the platinum group, as herein defined, may be selected from the group consisting of platinum, palladium, rhenium, iridium, rhodium, osmium, ruthenium or mixtures thereof, platinum being preferred. The metal of the tin group, as herein defined, may be selected from the group consisting of tin, germanium, lead or mixtures thereof, tin being preferred, which may be present as a compound such as the oxide. The promoter may be an alkaline metal selected from the group consisting of cesium, rubidium, potassium, sodium, lithium or mixtures thereof, or an alkaline-earth metal selected from the group consisting of baryum, strontium, calcium, magnesium or mixtures thereof, or even mixtures of alkaline and alkaline-earth metals, potassium being preferred ; other promoters known to be efficient may also be envisaged.

The alumina used as support generally has a specific area comprised between about 150 and 350 m²/g and a pore volume comprised between about 0.2 and 1.2 ml/g.

It is advisable to use an alumina having a purity higher than 98.5%, and in which the silica content does not exceed 1% while the iron content is lower than 0.1%.

The Applicant has unexpectedly found that the manner and the particular sequence of introduction of the catalytic metal compounds on the support, together with the conditions of intermediate calcination have an important influence on the catalyst activity in the scope of hydrocarbon dehydrogenation, hydrodrogenation or dehydrocyclisation reactions.

The Applicant has found that by applying the following sequence for the treatment of the alumina support, a suitable catalyst is obtained for use in processes such as the dehydrogenation of paraffinic hydrocarbons having from 3 to 6 carbon atoms, the dehydrocyclisation of hydrocarbons having a straight chain of at least 6 carbon atoms, or the hydrogenation of unsaturated hydrocarbons.

The alumina support is first impregnated with a compound of the metal of the tin group, in order to obtain a concentration of said metal in the final catalyst comprised between 0.1 and 2% by weight and preferably comprised between 0.15 and 1.0% by weight.

As metal of the tin group, it is preferable to use tin. According to an embodiment of the invention, a slurry is formed from alumina and an aqueous solution of tin chloride. Said slurry is dried at a temperature of about 80 to 100°C. The alumina cake which contains the tin chloride is then pulverised and is finally formed into pellets which are then calcined at a temperature comprised between about 450 and about 550°C during 12 to 20 hours.

The so-formed support is then treated so as to introduce therein a compound of the metal of the platinum group. According to the present invention, the support is treated so as to deposit thereon, in a first step, a small fraction of the total amount of the metal of the platinum group which has to be finally present. Generally, the amount of metal of the platinum group which is deposited at this stage is comprised between about 10% and about 40% of the total amount of the metal of the platinum group. The deposition of said metal may be carried out by means of any suitable technique such as impregnation. Generally, when platinum is to be deposited, the support is impregnated with an aqueous solution of chloroplatinic acid. After this impregnation, the newly impregnated support is submitted to a calcination at a temperature comprised between about 450 and about 550°C during 15 to 20 hours.

Thereafter, the calcined support is submitted to a reduction under hydrogen atmosphere at a temperature also comprised between about 450 and about 550°C, during from about 1 to about 4 hours.

The Applicant has found that this reduction step is very important and has a beneficial effect on the yield during the hydrogenation, dehydrogenation or dehydrocyclisation of hydrocarbons in the presence of this catalyst.

Before carrying out a second treatment with the metal of the platinum group, the support is submitted to an intermediate treatment with a promoter.

The alkaline or alkaline-earth metal is preferably well dispersed on the support. Generally the amount of alkaline or alkaline-earth metal compound present on the support is comprised between about 0.5 and about 5% by weight and more particularly between about 0.8 and 2.5% by weight expressed as metal. Preferably selected, potassium is most often deposited on the support in an amount corresponding to about 0.8 to about 2% by weight expressed as metal. It is believed that the promoter will generally be under the form of an oxide on the support rather than under its metallic form.

The promoter may be deposited on the support in accordance with any suitable method such as impregnation. It is however preferred to deposit the promoter, preferably potassium, by impregnation of the support with an aqueous solution of its nitrate.

The so-impregnated support is submitted to a calcination at a temperature comprised between about 380 and about 550°C in order to decompose the nitrate and to fix the oxide of alkaline or alkaline-earth metal on the support. Generally the calcination is carried out in air during about 3 to 7 hours.

3

According to the present invention, the metal of the platinum group is finally deposited on the so-formed support in an amount sufficient to have a total content of said metal on the support comprised between about 0.1 and about 2% by weight, and preferably between about 0.2 and 1% by weight. The deposit of this metal may be carried out by means of any suitable technique such as impregnation. However at this stage of treatment of the support, the removal of the compounds which have already been deposited thereon must be avoided. For this purpose, the Applicant has found that for this second impregnation of the metal of the platinum group, it is preferable to use an aqueous solution of a complex of platinum having the general formula $(Pt\ L_2)X_2$, $(Pt\ L_2)X_4$ or $(Pt\ L_4)X_2$, wherein L represents a ligand selected from $NH_3$, $R-NH_2$ or $NH_2-R-NH_2$, with R being an hydrocarbon radical having from 2 to 6 carbon atoms, preferably from 2 to 4 carbon atoms, and wherein X represents $NO_3$ or a halogen. An aqueous solution of the amminated complex having the formula $(Pt(NH_3)_4)Cl_2$ is most preferably used.

After this impregnation, the newly impregnated support is dried then submitted to a calcination in air at a temperature comprised between about 450° and 525°C during about 10 to 20 hours.

According to the present invention, the so-prepared catalyst may be used in a hydrocarbon dehydrocyclisation, hydrogenation and dehydrogenation process without necessitating the use of other promoters to increase the stability or the selectivity. The catalytic dehydrogenation of paraffinic hydrocarbons is a preferred use of the catalysts prepared according to the invention.

The process may be carried out in a fixed bed reactor or a mobile bed reactor or even a fluidised bed reactor; the process is most often carried out under continuous conditions, although it may also be carried out in batch. The hydrocarbon feed may be treated under the liquid form, or under a mixed phase liquid-vapour or also in vapour phase.

The effluent from the reaction zone contains a part of unconverted feed, hydrogen and reaction products. This effluent is first cooled and sent into a separator to separate hydrogen and to recover a liquid phase which is then submitted to a series of separations in order to recover the desired product, and to recycle the unconverted phase to the reactor.

In a dehydrogenation process, a feed of a paraffinic hydrocarbon having from 3 to 6 carbon atoms, preferably propane or any butane, most preferably propane, is contacted with the catalyst as hereabove described, under dehydrogenation operating conditions, generally in vapour phase.

The dehydrogenation process comprises the step of passing the feed to be dehydrogenated onto a catalyst prepared according to the invention, at a temperature comprised between about 400 and about 800°C, at a pressure comprised between about 1,013 and 1013,2 KPa (0.01 and 10 atm.) and at a weight hourly space velocity (WHSV) comprised between about 0.1 and 21.

More particularly, when propane is used as feed, the temperature is of about 530 to 650°C, the pressure is comprised between about 50.6 and 303.9 KPa (0.5 and 3 atm.) and the WHSV is comprised between about 1 and about 10.

The Applicant has unexpectedly found that by applying the process of the invention it becomes possible to considerably increase the yield of the dehydrogenation reaction when compared to the thermodynamic equilibrium, said yield being apt to exceed 90% when propane is dehydrogenated at a temperature of about 600°C.

The feed may be diluted with usual gaseous diluents, under molar ratios most often comprised between 1 and 10 mole of diluent per mole of feed. As diluent, $N_2$, $CO_2$ and $H_2O$ are the most often used.

The Applicant has also found that it is more interesting to co-feed hydrogen with the feed under molar ratios generally comprised between 0.05 and 0.5 mole of hydrogen per mole of feed, whether or not the feed is also diluted with gaseous diluents.

The catalyst prepared in accordance with the process of the invention is also suitable for the dehydrocyclization of straight chain acyclic hydrocarbons having in their structure a straight chain with at least 6 carbons atoms which may be cyclized. The preferred hydrocarbons are n-alkanes, although olefins may also be used. $C_6$-$C_{12}$ paraffins are generally used, and they will give benzene and/or lower alkyl aromatic compounds as the case may be, with a good yield and with a minimum of side reactions.

The dehydrocyclization in the presence of the catalyst of the invention may be carried out under the following operating conditions :

Temperature :        300-650°C
Pressure :           1-90 bar
LHSV :               0.1-20
Molar ratio $H_2/HC$ :   0.5 : 1 – 10 : 1 (HC = hydrocarbons)

According to another aspect of the present invention, there is provided a process to transfer hydrogen onto insatured hydrocarbons, said process consisting in contacting hydrocarbons with hydrogen under conversion conditions in the presence of a catalyst as hereabove described.

The hydrogenation process may be carried out under the following conditions :

Temperature : 100-400°C
Pressure : 1-90 bar
LHSV : 0.1-20
Molar ratio $H_2$/HC : 0.5 : 1 – 10 : 1

The following examples are given to better illustrate the process of the invention but without limiting its scope.

Example 1

Preparation of the catalyst.

A 99.8% purity alumina having a specific area of 189 $m^2$/g and a pore volume of 0.94 ml/g was used.

This alumina was first impregnated with an aqueous solution of $SnCl_2$. The impregnation was carried out in order to obtain a tin final content of 0.49% by weight. After this impregnation the support was calcined at 500°C during 18 hours.

The tin containing support was then impregnated with a solution of $H_2PtCl_6$ in order to deposit about 0.15% platinum on the support. The support was then submitted to a further calcination at 500°C during 18 hours. The support was let in the oven at 500°C and hydrogen was passed on the support during 1 hour in order to carry out a reduction.

The so-impregnated support was then treated with a solution of potassium nitrate in order to deposit thereon 1.00% by weight of potassium, expressed as metal.

After this impregnation, the support was then submitted to a calcination to fix the oxide of alkaline metal on the support ; said calcination was carried out in air at 400°C during 5 hours.

Finally, the second impregnation of the support with a platinum compound was carried out to obtain a platinum final content of 0.40% by weight. To carry out this impregnation the support was contacted with an aqueous solution of platinum amminated complex of formula $(Pt(NH_3)_4)Cl_2$. After the impregnation the support was calcined in air at 500°C during 18 hours.

A propane feed was dehydrogenated by passing it through a reactor in the presence of the catalyst hereabove prepared, and under the following operating conditions :

Temperature : 600°C
Pressure : 111.45 KPa (1.1 atm.)
WHSV : 3

Hydrogen was co-fed with the feed under a molar ratio of 0.1 mole/mole of feed.

Propylene was recovered in the effluent with a yield of 91.1% of the thermodynamic equilibrium under these conditions.

By way of comparison, different catalysts were prepared (Table I).

Catalyst A

Only one impregnation with platinum was carried out

Catalyst B

Only one impregnation with all the catalytic metals and the promoter was carried out

Catalyst C

A calcination and a reduction were carried out after the impregnation with platinum, but the second impregnation with platinum was not done.

Catalyst D

Platinum has been deposited through two impregnations, but without carrying out any calcination or reduction after the first impregnation.

Catalyst E

Platium has been deposited on a $Al_2O_3$/Sn support in a single impregnation, without adding a promoter.

Those catalysts were tested as hereabove described to dehydrogenate a propane feed in accordance with operating conditions identical to those hereabove described. The results are indicated in Table II.

EP 0 328 507 B1

Table I

| | Composition % | | | Impregnation SnCl₂ with calcination at 500°C | 1st impregnat. Pt | Calcination | Reduction | Impregnat. KNO₃ and calcin. at 400° C | 2nd impregnat. Pt and calcination |
|---|---|---|---|---|---|---|---|---|---|
| | Sn | Pt | K | | | | | | |
| Cat.A | 1.06 | 0.46 | 0.61 | yes | $H_2PtCl_6$ | 500°C | no | yes | no |
| Cat.B | 0.9 | 0.53 | 1.1 | only one impregnation with all the catalytic metals and calcination at 500°C | | | | | |
| Cat.C | 0.4 | 0.26 | 2.35 | yes | $H_2PtCl_6$ | drying 100°C | yes | yes | no |
| Cat.D | 1.01 | 0.83 | 2.11 | yes | $H_2PtCl_6$ (no calcination) | drying 100°C | no | drying 200°C | $(Pt(NH3)4)Cl_2$ 500°C |
| Cat.E | 0.98 | 0.49 | 0 | yes | $H_2PtCl_6$ | 500°C | no | no | no |

Table II

| | Yield/Theor. yield | Selectivity |
|---|---|---|
| Cat. A | 60% | 96.7% |
| Cat. B | 72% | 91.0% rapidly deactivating after 8 h |
| Cat. C | 83% | 95.9% |
| Cat. D | 60% | 96.7% |
| Cat. E | 69% | 93.0% |

EP 0 328 507 B1

### Examples 2 to 5

Different catalysts were prepared in accordance with the method of the invention (see Table III) with an alumina identical to that of Example 1.

### Table III

| | Composition% | | | Impregnation | Impregnation | Calcination | Reduction |
|---|---|---|---|---|---|---|---|
| | Pt | Sn | K | $SnCl_2$ + Calc. 500°C | Pt | | |
| 2 | 0.17 | 0.94 | 1.24 | yes | $H_2PtCl_6$ | 500°C | 1h/500°C |
| 3 | 0.74 | 1.00 | 0.9 | yes | $H_2PtCl_6$ | 480°C | 1h/500°C |
| 4 | 0.4 | 0.49 | 1.00 | yes | $H_2PtCl_6$ | 520°C | 1h/500°C |
| 5 | 0.83 | 0.95 | 1.41 | yes | $H_2PtCl_6$ | 500°C | 1h/500°C |

(continued)

| | Impregnation $KNO_3$ | Calcination | 2nd Impregnation Pt | Calcination |
|---|---|---|---|---|
| 2 | yes | 400°C | $(Pt(NH_3)_4)Cl_2$ | 500°C/18 h |
| 3 | yes | 370°C | $(Pt(NH_3)_4)Cl_2$ | 520°C/16 h |
| 4 | yes | 410°C | $(Pt(NH_3)_4)Cl_2$ | 500°C/20 h |
| 5 | yes | 400°C | $(Pt(NH_3)_4)Cl_2$ | 500°C/18 h |

These catalysts were used for the dehydrogenation of a propane feed in accordance with the following operating conditions.

T : 600°C
Pressure : 1.1 atm (0.11 MPa)
WHSV : 3
$H_2$/HC : 0.1 mole/mole (HC = hydrocarbons)

8

The results obtained with these catalysts are indicated in the following table.

## Table IV

| Examples | Yield/Theory | Selectivity |
| --- | --- | --- |
| 2 | 95.1% | 97.1% |
| 3 | 88.6% | 97 % |
| 4 | 92.8% | 98.5% |
| 5 | 95.3% | 97.2% |

### Example 6

The catalyst as prepared in Example 4 was used for the dehydrogenation of a n-butane feed under the following operating conditions.

T : 630°C
Pressure : 2 atm (0.2 MPa)
WHSV : 5
$H_2/HC$ : 0.4

The results obtained were the following

| Yield/Theory | Selectivity |
| --- | --- |
| 72% | 94.5% |

When a catalyst prepared as in the comparative example 1A, was used, a yield of only 48% was obtained with a selectivity of only 93.6%.

## Claims

1. Process for the preparation of a catalyst consisting of an alumina support containing at least one metal from the platinum group together with a co-catalyst and a promoter comprising the step of submitting the alumina support, containing the co-catalyst and calcined at a temperature comprising between 450 and 550°C,
   – to a first treatment with a compound of metal of the platinum group, said first treatment being followed by a calcination in air and by a reduction in the presence of hydrogen at a temperature comprised between 450 and 550°C ;
   – then to an intermediate treatment to deposit the promoter, said intermediate treatment being followed by a calcination at a temperature comprised between 380 and 550°C ;
   – and finally to a second treatment with a compound of metal of the platinum group, said second treatment being followed by a calcination at a temperature not exceeding 525°C,
   in order to obtain a catalyst containing from 0.1 to 2% by weight of at least one metal from the platinum group, from 0.1 to 2% by weight of at least one metal from the tin group as co-catalyst and from 0.5 to 5% by weight of at least one alkaline or alkaline-earth metal as promoter.

2. Process according to claim 1 wherein the catalyst contains from 0.15 to 1% by weight of a metal of the tin group, from 0.2 to 1% by weight from a metal of the platinum group, and from 0.8 to 2.5% by weight of an alkaline or alkaline-earth metal.

3. Process according to claim 1 or 2 wherein platinum is used as metal of the platinum group.

9

4. Process according to any one of claims 1 to 3 wherein the second impregnation with a metal of the platinum group is carried out with a compound of general formula $(Pt\ L_2)X_2$, $(Pt\ L_2)X_4$ or $(Pt\ L_4)X_2$ wherein L represents a ligand selected from the group comprising $NH_3$, $R\text{-}NH_2$ or $NH_2\text{-}R\text{-}NH_2$, with R being an hydrocarbon radical having from 2 to 6 carbon atoms and wherein X is $NO_3$ or a halogen.

5. Process according to any one of claims 1 to 4, wherein $(Pt(NH_3)_4)Cl_2$ is used as platinum compound for the second impregnation step.

6. Process according to any one of claims 1 to 5 wherein the promoter is potassium.

7. Process according to any one of claims 1 to 6, wherein the cocatalyst is tin.

8. Process for the dehydrogenation of $C_3$-$C_6$ aliphatic hydrocarbons which comprises the step of contacting a $C_3$-$C_6$ aliphatic hydrocarbon with a catalyst obtained in accordance with the process described in any one of claims 1 to 7, at a temperature comprised between about 400 and about 800°C, at a pressure comprised between about 0,1013 and about 1013,2 KPa (0.001 and about 10 atm.) and at a weight hourly space velocity comprised between about 0.1 and about 21.

9. Process for the dehydrogenation of propane which comprises the step of contacting propane with a catalyst obtained in accordance with the process of any one of claims 1 to 7, at a temperature of from about 530°C and about 650°C, under a pressure of from about 0.5 to about 3 bar, and with a weight hourly space velocity of from about 1 to 10.

10. Process according to claim 8 or 9, wherein about 0.05 to 0.5 mole of hydrogen is co-fed with each mole of hydrocarbon.

11. Process for the dehydrocyclization of aliphatic hydrocarbons having from 6 to 12 carbons atoms which comprises the step of contacting a $C_6$-$C_{12}$ n-alkane hydrocarbon with a catalyst obtained in accordance with the process of any one of claims 1 to 7, at a temperature comprised between about 300 and about 650°C, at a pressure comprised between about 1 and about 90 bars and at liquid hourly space velocity comprised between 0.1 and 20 $h^{-1}$, with a molar ratio $H_2$/Hydrocarbon comprised between 0.5 : 1 and 10 : 1.

12. Process for the hydrogenation of unsaturated compounds, which comprises the step of contacting an unsaturated compound with a catalyst obtained with the process described in any one of claims 1 to 7, at a temperature comprised between about 100 and about 400°C, under a pressure comprised between about 1 and about 90 bars and at a LHSV comprised between about 0.1 and about 20, with a molar ratio $H_2$/hydrocarbon comprised between about 0.5 : 1 and about 10 : 1.

13. Process for the catalytic dehydrogenation of propane, in the presence of hydrogen in a molar ratio of from 0.05 to 0.5 mole of hydrogen per mole of propane over a catalyst consisting of an alumina support containing at least one metal of the platinum group together with a co-catalyst and a promoter, which comprises the step of passing the feed to be dehydrogenated onto a catalyst containing from 0.2 to 1% by weight of platinum, from 0.15 to 1% by weight of tin as co-catalyst and from 0.8 to 2% by weight of potassium as promoter, said catalyst being obtained by submitting the alumina support containing the co-catalyst and calcined at a temperature comprised between 450 and 550°C,

– to a first treatment with a platinum compound, said first treatment being followed by a calcination in air and a reduction in the presence of hydrogen at a temperature comprised between 450 and 550°C ;

– then to an intermediate treatment to deposit potassium, said intermediate treatment being followed by a calcination at a temperature comprised between 380 and 550°C,

– and finally to a second treatment with a platinum compound, said second treatment being followed by a calcination at a temperature not exceeding 525°C,

the dehydrogenation being carried out in the presence of said catalyst at a temperature comprised between 530°C and 650°C, a pressure comprised between 50,6 and 303,9 kPa (0.5 and 3 atm.) and a weight hourly space velocity comprised between 1 and 10.

## Ansprüche

1. Verfahren zur Herstellung eines aus einem Alumiumoxid-Träger bestehenden Katalysators, der mindestens ein Metall aus der Platin-Gruppe zusammen mit einem Cokatalysator und einem Beschleuniger enthält, das die Schritte aufweist, daß der den Cokatalysator enthaltende und bei einer Temperatur zwischen 450 und 550°C kalzinierte Aluminiumoxid-Träger

– einer ersten Behandlung mit einer Metallverbindung der Platin-Gruppe unterworfen wird, wobei die erste Behandlung von einer Kalzinierung in Luft und von einer Reduktion in der Gegenwart von Wasserstoff bei einer Temperatur zwischen 450 und 550°C gefolgt wird ;

– dann einer Zwischenbehandlung unterworfen wird, um den beschleuniger niederzuschlagen, wobei die Zwischenbehandlung von einer Kalzinierung bei einer Temperatur zwischen 380 und 550°C gefolgt wird ;

– und zum Schluß einer zweiten Behandlung mit einer Metallverbindung der Platin-Gruppe unterworfen wird, wobei die zweite Behandlung von einer Kalzinierung bei einer Temperatur gefolgt wird, die 525°C nicht übersteigt,

um einen Katalysator zu erhalten, der 0,1 bnis 2 Gew.-% mindestens eines Metalls aus der Platin-Gruppe, 0,1 bis 2 Gew.-% mindestens eines Metalls aus der Zinn-Gruppe als Cokatalysator und 0,5 bis 5 Gew.-% mindestens eines Alkali- oder Erdalkalimetalls als Beschleuniger enthält.

2. Verfahren nach Anspruch 1, wobei der Katalysator 0,15 bis 1 Gew.-% eines Metalls aus der Zinn-Gruppe, 0,2 bis 1 Gew.-% eines Metalls aus der Platin-Gruppe und 0,8 bis 2,5 Gew.-% eines Alkali- oder Erdalkalimetalls enthält.

3. Verfahren nach Anspruch 1 oder 2, wobei Platin als Metall aus der Platin-Gruppe verwender wird.

4. Verfahren nach einem der Ansprüche 1 bis 3, wobei die zweite Imprägnierung mit einem Metall aus der Platin-Gruppe mit einer Verbindung der allgemeinen Formel $(Pt\ L_2)X_2$, $(Pt\ L_2)X_4$ oder $(Pt\ L_4)X_2$ ausgeführt wird, wobei L einen Liganden darstell, der aus $NH_3$, $R-NH_2$ oder $NH_2-R-NH_2$ ausgewählt ist, wobei R ein Kohlenwasserstoffrest mit 2 bis 6 Kohlenstoffatomen ist und wobei X $NO_3$ oder ein Halogen ist.

5. Verfahren nach einem der Ansprüche 1 bis 4, wobei $(Pt(NH_3)_4)Cl_2$ als Platinverbindung für den zweiten Imprägnierungsschritt verwendet wird.

6. Verfahren nach einem der Ansprüche 1 bis 5, wobei der beschleuniger Kalium ist.

7. Verfahren nach einem der Ansprüche 1 bis 6, wobei der Cokatalysator Zinn ist.

8. Verfahren zur Dehydrierung von $C_3$-$C_6$-aliphatischen Kohlenwasserstoffen, das den Schritt des Kontaktierens eines $C_3$-$C_6$-aliphatischen Kohlenwasserstoffs mit einem Katalysator, der mit einem Verfahren nach einem der Ansprüche 1 bis 7 erhalten wurde, bei einer Temperatur zwischen etwa 400 und etwa 800°C, einem Druck zwischen etwa 0,1013 und etwa 1013,2 kPa (0,001 und 10 atm) und bei einer stündlichen Gewichtstraumgeschwindigkeit zwischen etwa 0,1 und etwa 21, aufweist.

9. Verfahren zur Dehydrierung von Propan, das den Schritt des Kontaktierens von Propan mit einem Katalysator, der mit einem Verfahren nach einem der Ansprüche 1 bis 7 erhalten wurde, bei einer Temperatur von etwa 530°C und etwa 650°C, unter einem Druck von etwa 0,5 bis etwa 3 bar und mit einer stündlichen Gewichtsraumgeschwindigkeit von etwa 1 bis 10, aufweist.

10. Verfahren nach Anspruch 8 oder 9, wobei etwa 0,05 bis 0,5 Mol Wasserstoff mit jedem Mol Kohlenwasserstoff eingespeist werden.

11. Verfahren zur Dehydrocyklisierung von aliphatischen Kohlenwasserstoffen mit 6 bis 12 Kohlenstoffatomen, das den Schritt des Kontaktierens eines $C_6$-$C_{12}$-n-Alkan-Kohlenwasserstoffs mit einem Katalysator, der mit einem Verfahren nach einem der Ansprüche 1 bis 7 erhalten wurde, bei einer Temperatur zwischen etwa 300 und etwa 650°C, bei einem Druck zwischen etwa 1 und etwa 90 bar und bei einer stündlichen Flüssigkeitsraumgeschwindigkeit zwischen 0,1 und 20 h$^{-1}$, mit einem Molverhältnis $H_2$/Kohlenwasserstoff zwischen 0,5 : 1 und 10 : 1, aufweist.

12. Verfahren zur Hydrierung von ungesättigten Verbindungen, das den Schritt des Kontaktierens einer ungesättigten Verbindung mit einem Katalysator, der mit einem Vefahren nach einem der Ansprüche 1 bis 7 erhalten wurde, bei einer Temperatur zwischen etwa 100 und etwa 400°C, unter einem Druck zwischen etwa 1 und etwa 90 bar und bei einer LHSV zwischen etwa 0,1 und etwa 20, mit einem Molverhählnis $H_2$/Kohlenwasserstoff zwischen etwa 0,5 : 1 und etwa 10 : 1, aufweist.

13. Verfahren zur katalytischen Dehydrierung von Propan, in der Gegenwart von Wasserstoff in einem Molverhältnis von 0,05 bis 0,5 Mol Wassertoff pro Mol Propan, über einem Katalysator, der aus einem Aluminiumoxid-Träger besteht, der mindestens ein Metall aus der Platin-Gruppe zusammmen mit einem Cokatalysator und einem Beschleuniger enthält, das die Schritte aufweist, daß das zu dehydrierende Einsatzmaterial auf einem Katalysator geleitet wird, der 0,2 bis 1 Gew.-% Platin, 0,15 bis 1 Gew.-% Zinn als Cokatalysator und 0,8 bis 2 Gew.-% Kalium als Beschleuniger enthält, wobei der Katalysator erhalten wird, in dem den Cokatalysator enthaltende und bei einer Temperatur zwischen 450 und 550°C kalzinierte Aluminiumoxid-Träger

– einer ersten Behandlung mit einer Platin-Verbindung unterworfen wird, wobei die erste Behandlung von einer Kalzinierung in Luft und einer Reduktion in der Gegenwart von Wasserstoff bei einer Temperatur zwischen 450 und 550°C gefolgt wird ;

– dann einer Zwischenbehandlung unterworfen wird, um Kalium niederzuschlagen, wobei die Zwischenbehandlung von einer Kalzinierung bei einer Temperatur zwischen 380 und 550°C gefolgt wird,

– und zum Schluß einer zweiten Behandlung mit einer Platinverbindung unterworfen wird, wobei die zweite Behandlung von einer Kalzinierung bei einer Temperatur gefolgt wird, die 525°C nicht übersteigt,

wobei die Dehydrierung in der Gegewart des Katalysators bei einer Temperatur zwischen 530°C und 650°C, einem Druck zwischen 50,6 und 303,9 kPa (0,5 und 3 atm) und bei einer stündlichen Gewichtsraumgeschwindigkeit zwischen 1 und 10 ausgeführt wird.

## Revendications

1. Procédé de préparation du catalyseur sur support d'alumine contenant au moins un métal du groupe du platine ainsi qu'un co-catalyseur et un promoteur, caractérisé en ce que l'on soumet le support d'alumine contenant le co-catalyseur et calciné à une température comprise entre 450 et 550°C :

– à un premier traitement avec un composé de métal du groupe du platine, ce premier traitement étant suivi d'une calcination à l'air et d'une réduction en présence d'hydrogène à une température comprise entre 450 et 550°C,

– puis à un traitement intermédiaire pour déposer le promoteur, ce traitement intermédiaire étant suivi d'une calcination à une température comprises entre 380 et 550°C,

– et enfin à un second traitement avec un composé de métal du groupe du platine, ce second traitement étant suivi d'une calcination à une température ne dépassant pas 525°C,

afin d'obtenir un catalyseur contenant de 0,1 à 2% en poids d'au moins un métal du groupe du platine, de 01, à 2% en poids d'au moins un métal du groupe de l'étain comme co-catalyseur et de 0,5 à 5% en poids d'un métal alcalin ou alcalino-terreux comme promoteur.

2. Procédé selon la revendication 1 caractérisé en ce que le catalyseur contient de 0,15 à 1% en poids d'un métal du groupe de l'étain, de 0,2 à 1% en poids d'un métal du groupe de platine, et de 0,8 à 2,5% en poids d'un métal alcalin ou alcalino-terreux.

3. Procédé selon l'une quelconque des revendications 1 et 2, caractérisé en ce que l'on utilise le platine comme métal du groupe du platine.

4. Procédé selon l'une quelconque des revendications 1 à 3 caractérisé en ce que l'on utilise lors de la seconde imprégnation avec un métal du groupe du platine, un composé de formule générale $(PtL_2) X_2$, $(PtL_2) X_4$ ou $(PtL_4) X_2$ dans laquelle L représente un ligand choisi parmi $NH_3$, $R-NH_2$ ou $NH_2-R-NH_2$, avec R étant un radical hydrocarboné ayant de 2 à 6 atomes de carbone et où X représente $NO_3$ ou un halogène.

5. Procédé selon l'une quelconque des revendications 1 à 4, caractérisé en ce que l'on utilise $Pt(NH_3)_4CL_2$ comme composé du platine lors de la seconde étape d'imprégnation.

6. Procédé selon l'une quelconque des revendications 1 à 5, caractérisé en ce que le promoteur est le potassium.

7. Procédé selon l'une quelconque des revendications 1 à 6 caractérisé en ce que le cocatalyseur est l'étain.

8. Procédé de déshydrogénation d'hydrocarbures aliphatiques en $C_3-C_6$, caractérisé en ce qu'il consiste à mettre en contact un hydrocarbure aliphatique en $C_3-C_6$ avec un catalyseur obtenu avec le procédé suivant l'une des revendications 1 à 7 à une température comprise entre 400 et 800°C, sous une pression comprise entre 0,1013 et 1013,2 kPa (0,001 et 10 atm.) et à une vitesse spatiale horaire en poids comprise entre 0,1 et 21.

9. Procédé de déshydrogénation du propane, caractérisé en ce qu'il consiste à mettre en contact du propane avec un catalyseur obtenu avec le procédé suivant l'une des revendications 1 à 7 à une température comprises entre 530 et 650°C, sous une pression comprise entre 0,5 et 3 bars et à une vitesse spatiale horaire en poids comprises entre 1 et 10.

10. Procédé selon l'une quelconque des revendications 8 à 9, caractérisé en ce que de 0,05 à 0,5 mole d'hydrogène est introduit conjointement avec chaque mole d'hydrocarbure.

11. Procédé de déshydrocylisation d'hydrocarbures aliphatiques en $C_6-C_{12}$ caractérisé en ce qu'il consiste à mettre en contact un hydrocarbures n-alcane en $C_6-C_{12}$ avec un catalyseur obtenu selon le procédé suivant l'une des revendications 1 à 7, à une température comprises entre 300 et 650°C, sous une pression comprise entre 1 et 90 bars, à une vitesse spatiale horaire liquide (LHSV) comprise entre 0,1 et 20 $h^{-1}$ et avec un rapport molaire $H_2$/hydrocarbure compris entre 0,5 : 1 et 10 : 1.

12. Procédé d'hydrogénation de composés insaturés, caractérisé en ce qu'il consiste à mettre en contact un composé insaturé avec un catalyseur obtenu avec le procédé suivant les revendications 1 à 7 à une température comprise entre 100 et 400°C, sous une pression de 1 à 90 bars, à une LHSV comprises entre 0,1 et 20 et avec un rapport molaire $H_2$/hydrocarbure compris entre 0,5 : 1 et 10 : 1.

13. Procédé de déshydrogénation catalytique du propane en présence d'hydrogène dans un rapport molaire de 0,05 et 0,5 mole d'hydrogène par mole de propane sur un catalyseur consistant en un support d'alumine contenant au moins un métal du groupe du platine ainsi qu'un co-catalyseur et un promoteur, caractérisé en ce que l'on fait passer la charge à déshydrogéner sur un catalyseur contenant de 0,2 à 1% en poids de platine, de 0,15 à 1% en poids d'étain comme co-catalyseur et de 0,8 à 2% en poids de potassium comme promoteur, ce catalyseur étant obtenu en soumettant le support d'alumine contenant le co-catalyseur et calciné à une température comprise entre 450 et 550°C :

– à un premier traitement avec un composé de platine, ce premier traitement étant suivi d'une calcination à l'air et d'une réduction en présence d'hydrogène à une température comprise entre 450 et 550°C,

– puis à un traitement intermédiaire pour déposer le potassium, ce traitement intermédiaire étant suivi d'une calcination à une température comprise entre 380 et 550°C,

– et, enfin, à un second traitement avec un composé de platine, ce second traitement étant suivi d'une calcination à une température ne dépassant pas 525°C,

la déshydrogénation s'effectuant en présence de ce catalyseur à une température comprise entre 530 et 650°C, une pression comprise entre 50,6 et 303,9 kPa (0,5 et 3 atm.) et une vitesse spatiale horaire en poids comprise entre 1 et 10.